(19) Europäisches Patentamt

European Patent Office

Office européen des brevets

(11) **EP 4 252 641 B1**

(12) **EUROPEAN PATENT SPECIFICATION**

(45) Date of publication and mention
of the grant of the patent:
**23.10.2024 Bulletin 2024/43**

(21) Application number: **22166395.8**

(22) Date of filing: **01.04.2022**

(51) International Patent Classification (IPC):
***A61B 5/08*** *(2006.01)* ***A61B 5/00*** *(2006.01)*
***A61B 5/11*** *(2006.01)*

(52) Cooperative Patent Classification (CPC):
**A61B 5/0816; A61B 5/1118; A61B 5/6801;**
**A61B 5/7246; A61B 5/725; A61B 5/7264;**
A61B 2562/0219

(54) **RESPIRATION RATE MONITORING SYSTEM AND METHOD**

SYSTEM UND VERFAHREN ZUR ÜBERWACHUNG DER ATMUNGSRATE

SYSTÈME ET PROCÉDÉ DE SURVEILLANCE DE FRÉQUENCE RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**04.10.2023 Bulletin 2023/40**

(73) Proprietor: **Sensium Healthcare Limited
Abingdon, Oxfordshire OX14 4SA (GB)**

(72) Inventor: **Burdett, Alison
Oxford, OX2 0AU (GB)**

(74) Representative: **Marks & Clerk LLP
15 Fetter Lane
London EC4A 1BW (GB)**

(56) References cited:
WO-A1-2016/085879    US-A1- 2015 112 605
US-A1- 2018 056 128    US-A1- 2020 163 586
US-A1- 2020 253 509

## Description

Field of the Invention

The present invention relates to a method for identifying a validly determined respiration rate, a respiration rate monitoring system, and one or more processors for use therewith.

Background

A cause of sub-optimal care in hospitals is that indications of clinical deterioration are not appreciated or not acted upon in time by clinicians. Vital signs are typically recorded by nurses at discrete points in time during a shift resulting in gaps in the monitoring of patients. Adverse physiological events flagging patient deterioration may occur at a time when a patient's vital signs are not being monitored and may only be picked up later when the vital signs are next checked.

Monitoring patients continuously, so that the appropriate level of response can be quickly administered to prevent patient deterioration following early warning signs, assists in the rapid detection of adverse physiological events.

EP1928311 describes a low cost, lightweight and unobtrusive wireless digital plaster that continuously monitors heart rate (HR), respiration activity and temperature. Thereby, physiological data from patients are transmitted via hotspots (bridges) to one or more servers that enable further analysis and presentation of the values and trends of vital signs in computer monitors and mobile devices. The servers are programmed to generate notifications when the nominal values of one or more of these parameters have exceeded pre-set limits and deliver these notifications via central stations and/or mobile devices. Thus, the medical staff are warned about adverse physiological events that may lead to posterior deterioration of the patient if left untreated.

The majority of patients in a general ward are not stationary and will move around and perform physical actions such as walking, eating, drinking, speaking, and coughing and so on. This introduces an additional challenge to early warning monitoring technologies. Motion of a patent often results in artefacts contaminating the physiological signals obtained from that patient, owing to electrical signals generated by movement of the patient's muscles, expansion and contraction of the lungs and so on. Such noise in a physiological signal not only affects the quality and/or reliability of the processed physiological values but may also incorrectly be identified as an adverse physiological event and result in false alarms.

EP2677927 proposes a respiration monitoring method and system using impedance pneuomography. Impedance pneumography is a method of obtaining a respiration waveform of a patient by measuring changes in thoracic impedance between two ECG electrodes as the patient breaths. Thoracic impedance changes with the expansion and contraction of the patient's lungs during inhalation and exhalation so measuring thoracic impedance provides an indication of the patient's respiration rate. However, thoracic impedance can also vary due to other physical movements of the patient that cause changes in the physiology of the thorax such as movement, coughing or even the presence of a heartbeat. Other activities such as talking, sighing, eating and drinking can also lead to very irregular impedance pneumography waveforms. These noisy and irregular signals can mask the impedance changes arising from the patient's respiration and may thus result in an incorrect estimate of respiration rate that leads to incorrect identification of adverse physiological events and false alarms.

When a monitoring system consistently generates false alarms, clinicians may become desensitised to such alarms as they attribute the most likely cause to be noise rather than an actual adverse physiological event. This reduces clinicians' trust in such monitoring systems despite their potential to improve patient safety and may even cause a clinician to interpret a genuine alarm as a false one.

Further, if the monitoring system is used for collecting data sets, for example or subsequent analysis and research purposes rather than for an alarm or early warning system, it may be difficult to determine which parts of the data are unreliable or incorrect respiration rate estimates and which are accurate estimates.

US2020/0163586A1 discloses a monitoring apparatus for measuring the respiratory rate of a subject and comprising a sensor for monitoring blood volume and a movement sensor. The sensed data is used to determine a respiration rate with higher or lower confidence.

Summary

According to a first aspect of the invention, there is provided a computer-implemented method of identifying a validly determined respiration rate, the method comprising: determining a respiration rate and a confidence level associated with the respiration rate from a waveform signal representative of a recorded respiration episode; associating the respiration rate with one of at least first and second classes, the classes being associated with respective different confidence level thresholds; wherein at least one of the classes is a first range of respiration rates, wherein at least one of the classes is a second range of respiration rates, wherein the first range comprises a normal respiration rate range of a patient and the second range comprises a respiration rate range outside said normal respiration rate range and wherein the confidence level threshold associated with the first class is lower than that associated with the second class and if the confidence level exceeds a confidence level threshold associated with the class with which the respiration rate is associated, determining that the respiration rate is valid and outputting the determined respiration rate; or if the confidence level is below the confidence level threshold associated with the class with which the respiration rate is associated, determining that the respiration rate is invalid and outputting an invalid result notification.

Optionally, determining the confidence level comprises applying a logistic regression-based model to the waveform signal.

Optionally, determining the respiration rate comprises applying a rules-based model to the waveform signal to identify peaks and troughs in the waveform signal, and determining the respiration rate from a periodicity of the identified peaks and troughs.

Optionally, at least one of the classes is a first physical activity level of the patient, wherein at least one of the classes is a second physical activity level of the patient, and wherein said associating the respiration rate with the one of at least first and second classes comprises: applying a classifier to accelerometer data captured from the patient during said respiration episode to classify the patient's physical activity during said respiration episode into said first or second activity level classes, and associating the respiration rate of said respiration episode with said first or second activity level classes.

Optionally, at least one of the classes is an expected respiration rate range, wherein at least one of the classes is an unexpected respiration rate range, and wherein said associating the respiration rate with the one of at least first and second classes comprises: applying a Kalman filter to previously determined respiration rates to determine a range of expected and unexpected respiration rates, and determining if the respiration rate is in the expected or unexpected respiration rate range.

According to a second aspect of the invention, there is provided one or more processors for a respiration monitoring system, the one or more processors configured for: (i) determining a respiration rate and a confidence level associated with the respiration rate from a waveform signal representative of a recorded respiration episode; (ii) associating the respiration rate with one of at least first and second classes, the classes being associated with respective different confidence level thresholds wherein at least one of the classes is a first range of respiration rates, wherein at least one of the classes is a second range of respiration rates, wherein the first range comprises a normal respiration rate range of a patient and the second range comprises a respiration rate range outside said normal respiration rate range and wherein the confidence level threshold associated with the first class is lower than that associated with the second class; and (iii) if the confidence level exceeds a confidence level threshold associated with the class with which the respiration rate is associated, determining that the respiration rate is valid and outputting the determined respiration rate; or (iv) if the confidence level is below the confidence level threshold associated with the class with which the respiration rate is associated, determining that the respiration rate as invalid and outputting an invalid result notification.

According to a third aspect of the invention, there is provided a respiration monitoring system comprising: a body-worn sensor for recording a waveform signal representative of a respiration episode; and one or more processors according to the above second aspect.

Optionally, the one or more processors performing the steps of determining the respiration rate and determining the confidence level are co-located with the body-worn sensor.

Optionally, the system comprises an accelerometer co-located with the body-worn sensor and configured to record accelerometer data during said respiration episode.

Optionally, the system comprises a transmitter co-located with the body-worn sensor and configured to transmit the determined respiration rate and confidence level, and where applicable accelerometer data, to a server remote from the body-worn sensor.

Optionally, the body-worn sensor, the one or more co-located processors and where applicable the co-located transmitter and co-located accelerometer are configured as a wearable wireless device and/or a low-power battery operated disposable device.

Brief Description of the drawings

Figure 1 is a flow diagram for a computer-implemented method of the invention.

Figure 2 is a block diagram of a respiration rate monitoring system according to the invention.

Figure 3 is a block diagram of a respiration rate monitoring system according to the invention.

Figure 4 is a block diagram of a respiration rate monitoring system according to the invention.

Detailed Description

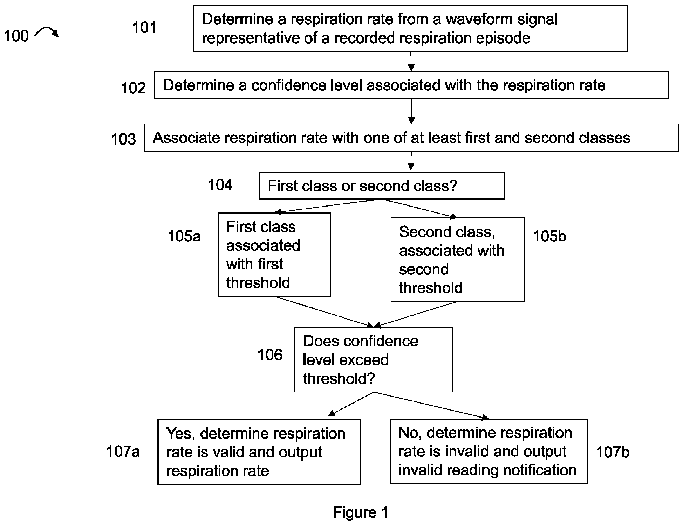

Figures 1 is a flow diagram for a computer-implemented method 100 of identifying a validly determined respiration rate. In general terms, the method 100 uses different confidence level thresholds depending on what class the determined respiration rate is associated with. The classes may be identified from the value of the determined respiration rate but also or alternatively from other factors. For example, one or more features of the respiration waveform signal, one or more previous, historical respiration rate readings of a given patient, accelerometer data from the patient and other data may indicate it is beneficial to apply a different threshold to the confidence level to identify if a reading is valid or not. If the confidence level of a measured respiration rate is at or above the applied threshold, the respiration rate is considered a valid reading whereas if it is below the applied threshold, it is considered invalid.

For example, consider the non-limiting example of where the confidence level threshold depends on which range class the value of the determined respiration rate is assigned. The threshold of whether or not to accept the reading as valid is effectively adapted according to the potential seriousness of an adverse physiological event. That is, the further away a measured respiration rate is from a normal range, the higher the confidence level that reading needs to have to be considered a valid reading. In this way, highly abnormal respiration rate measurements that are likely to require a clinician's attention should they be valid, will only be deemed valid if they actually arise from high-quality, low noise respiration signals. Conversely, respiration rate measurements that fall within the normal range so would unlikely require a clinician's attention need not have as high a confidence level.

Not only does this reduce the number of false alarms but it also overcomes a problem that using a single, high-confidence threshold might have. Namely, if there is only a single threshold which is set to be very high (e.g. 90%-100%) to reduce false alarms, too few readings are deemed valid. This causes the monitoring system to output readings infrequently or sporadically because most of the readings are under the threshold. In turn, this may mean adverse physiological events are missed and/or if the collected data is being used for subsequent analysis or research, there may be large gaps in the data. In contrast, the multi-threshold approach described herein ensures there is continuous and regular monitoring as the threshold is lowered (e.g. to around 50%) when respiration rate readings are normal or expected for a given patient. For example because, based on previously recorded data, the respiration rate readings are in a predetermined normal respiration rate range.

Consider an alternative scenario where the patient's respiration rate value is similar to the values recorded during a previous period of time (e.g. within a few breaths per minute of the mean of the previous 15 minutes). The similarity means the new measurement at or around the same value is expected and unlikely to be an outlier caused by noise, and so a relatively low confidence level threshold can be applied. Conversely, if the recorded respiration rate value is very different to the previous values, then either it is an outlier caused by noise or it is indicative of an adverse physiological event requiring a clinician's attention. In such a case, the confidence level threshold could be raised for just the dissimilar reading and the reading would only be identified as valid if the associated confidence level meets the increased threshold.

Again, in a further scenario, a patient is likely to move around and be active (e.g. walk, cough, speak etc.) at times. Respiration rate readings obtained while a patient has a high activity level are likely to suffer from motion artefacts. In such a case, accelerometer data may be obtained during the recorded respiration episode and fed into a classifier of the monitoring system to classify the activity level and thus the associated respiration rate recorded during that time into e.g. the classes of no activity, low activity or moderate activity. The likelihood of motion artefacts during low activity levels is small so a low confidence level threshold may be applied. Conversely, higher confidence level thresholds may be applied to higher activity levels.

As will be appreciated, the above examples are intended to be illustrative.

Accordingly, referring to Figure 1, the method 100 comprises determining 101 a respiration rate from a waveform signal representative of a recorded respiration episode and determining 102 a confidence level associated with the respiration rate. The determined respiration rate is then associated with 103 one of at least first and second classes, the classes being associated with respective different confidence level thresholds. If 104 the waveform signal is associated with a first class, the method 100 comprises applying 105a a first threshold value to the confidence level. Conversely, if 104 the waveform signal is classified into a second class, the method 100 comprises applying 105b a second, higher threshold to the confidence level. If 106 the confidence level is above the applied first or second threshold value as applicable, the method 100 comprises determining 107a that the respiration rate is valid and outputting the determined respiration rate. If 105 the confidence level is below the applied first or second threshold value as applicable, the method comprises determining 107b that the respiration rate is invalid. In this scenario, an error code, message or other indication that the determined respiration rate is invalid may be output instead. As described above, the classes used may be derived from any input to the monitoring system. A non-exhaustive list of such inputs includes: one or more ranges of respiration rates, similarity to previously recorded data, accelerometer data, one or more sets of features of the waveform signal such as peaks, troughs or zero-crossings, patient history and/or other such data.

It is envisaged that the step of determining 101 the respiration rate may be performed according one or more of the methods described in EP2677927. That is, a rules-based algorithm may be applied in the time-domain to the waveform signal obtained via impedance pneumography to identify peaks and troughs, and various derivatives thereof, and used to determine a periodicity in the waveform signal that is indicative of the respiration rate.

It is envisaged that the step of determining 102 the confidence level associated with the respiration rate is to apply a logistic regression-based model to the recorded waveform signal and/or a set of low-level features thereof. That is, a logistic regression-based classifier may be trained to output a probability i.e. a confidence level that a waveform signal from which the respiration rate has been determined is a high quality, low noise recording. An example implementation of a logistic regression classifier that may be used for this purpose will now be described.

The confidence level, also described herein as a signal quality score (SQS) or probability p, is an indicator of how "clean" the input respiration waveform is. As described above, the respiration waveform may be obtained via impedance pneumography, which measures changes in thoracic impedance between the two ECG electrodes. The thoracic impedance changes with the expansion and contraction of the lungs during inhalation and exhalation. However thoracic impedance can also vary due to physical movement as well as any other activity that brings changes in the physiology of the thorax such as eating, drinking, coughing and heart beating. These "contaminant" signals can mask the impedance changes due to respiration and result in the calculation of an incorrect RR. Therefore, the SQS aims to identify such contaminant waveforms via a low score, while clean and regular waveforms would score highly. The SQS is a proxy to determine whether the determined respiration rate is accurate or not. The SQS will be represented as a real number between 0 (lowest) and 1 (highest). Effectively the SQS is a value of the probability *'p'* with which the respiration signal is *likely to be noise free* thus affirming the determined respiration rate is accurate and thus valid.

Signal quality is measured using certain specific attributes of the respiration signal. Assume that we have respiration signals which are indexed from 0,1,2, ... , $N$ - 1, and that from each of the $N$ signals, we generate *'n'* attributes such as $x_0$, $x_1$, ..., $x_n$.

A simple way to produce an SQS from these attributes would be to define the SQS to be a linear combination of the attributes:

$$SQS = \beta \mathbb{x} + \beta_0 \qquad (1)$$

Where $\beta_0$ is the intercept, $\beta$ is the slope of the linear equation and $\mathbb{x}$ is a vector of all the attributes. Since the respiration signals are real signals, the attributes generated from the signals are also real but discrete. This means that the range of each of the attributes spans the entire real line from $-\infty$ to $+\infty$, and from equation (1) would result in a value of SQS whose range would span the entire real line. This is not acceptable as we have defined the SQS to be a number between 0 and 1. Therefore a linear combination of attributes which produces an unbounded output is not a good hypothesis function which can represent the SQS.

Another approach would be to define the SQS as the log of the linear combination of the features:

$$SQS = \log(\beta \mathbb{x} + \beta_0) \qquad (2)$$

The problem with this approach is that the log of the linear combination of real discrete numbers would be bounded to 0 on the left but would be unbounded in the positive direction of the real line. This would again be unacceptable.

In order to define the SQS such that the value is bounded in both the positive and the negative direction of the real line, we simply consider the logistic transformation of the SQS and let that transformed value be the linear combination of the attributes of the signal. This is defined as below.

$$log \left( \frac{SQS}{1-SQS} \right) = \beta \mathbb{x} + \beta_0 \qquad (3)$$

Solving the above equation (3) for SQS, we get:

$$SQS = \frac{1}{1+e^{-(\beta x + \beta_0)}} \qquad (4)$$

That is, we get a sigmoid function that accordingly guarantees the output value for SQS is always a number in the range between 0 and 1.

Sigmoid functions have uses as the activation function of a number of different classifiers. In this case, the sigmoid corresponds to the activation stage of a generalised, linear, logistic regression model. As will be appreciated, logistic regression is a discriminative model. As opposed to a generative model that relies on prior probabilities when making decision about the probability of occurrence of a certain event, the discriminative models work by choosing an event that maximises the probability of the observed data. In this model, we assume that the log odds of the probability varies proportional to the linear combination of the attributes instead of assuming that the probability itself varies according to the linear combination of the input attributes.

As described above, the aim of the confidence level or SQS is to decide whether to output (e.g. display) a given respiration rate value or to determine it is invalid. As will be appreciated, the sigmoid function in equation (4) is odd at the point where the SQS or probability $p$ is equal to 0.5. Thus, one approach would be to select a confidence level threshold value of 0.5. Respiration rates with confidence levels at or above this value would be deemed valid is deemed valid, those with confidence levels below it invalid.

This approach may be described as classification of signals into two classes or groups (valid and invalid) and can be modelled using known machine learning classification models. Note that whist the term classification is used, the purpose of the classifier in this context is primarily to obtain the confidence level value of the respiration rate determined from the waveform signal using the separate, rules-based approach, rather than to actually classify the determined respiration rate itself into valid or invalid. The final determination of valid or invalid reading is made by applying different thresholds to obtained SQS.

Returning to the method of obtaining the SQS, formally, a binary response variable is called an indicator variable. Let the response or the classification be defined as which can take on two values 0 and 1. Thus writing this in an equation form, we get:

$$C = \begin{cases} 1 \ SQS \ \geq 0.5 \\ 0 \ SQS \ < 0.5 \end{cases} \qquad (5)$$

Since we have already said that the value of SQS is the value of the probability, the above equation can be written in terms of probability $p$ as below:

$$C = \begin{cases} 1 \ p \ \geq 0.5 \\ 0 \ p \ < 0.5 \end{cases} \qquad (6)$$

Equation (4) i.e. the sigmoid function can now be compactly defined in terms of conditional probabilities as:

$$\Pr(C = 1|\mathbb{X}) = 1 \ \text{ and } \Pr(C = 0|\mathbb{X}) = 0 \qquad (7)$$

In the above equations, $\mathbb{X}$ is a vector of attributes that are generated from the respiration waveform signals, as will be described in more detail below. There is one vector $\mathbb{X}$ for every respiration signal. The conditional probability in equation (7) represents the probability of occurrence of class = 1 given a signal (data) represented by its attribute vector $\mathbb{X} = [x_0, x_1]$.

The hypothesis function that maps our input attribute vector into an output response variable is the SQS defined in equation (4).

Because the classes, C = 1 and C = 0 represent two realisations of a Bernoulli random variable C, if the probability of a certain input respiration signal belonging to class C = 1 is $p$, then the probability of the same input signal belonging to class C= 0 is 1 - $p$.

In a Machine Learning framework, the available data which is in the form of attribute representation is split into training, testing and validation sets. This data is completely defined by a set of attributes such as and a target variable which represents the value of response for that signal. The rationale behind fitting a model (such as the classification model described above) to the data (which encompass attributes and response value) is to minimise the error between the expected classification labels and those generated by the trained model. Thus, the learning method we followed here corresponds to a supervised learning approach. Since the selected model produces probabilities and not a quantitative value, in order to minimise the error in probabilities, we need to define a likelihood or a log likelihood function. The likelihood for a Bernoulli random variable is defined as below.

$$l(\beta_0, \beta) = \prod_{i=1}^{N} p(x_i)^{C_i} (1 - p(x_i))^{1-C_i} \tag{8}$$

The log likelihood is then of the form:

$$l(\beta_0, \beta) = \sum_{i=1}^{N} C_i \log p(x_i) + (1 - C_i) \log 1 - p(x_i) \tag{9}$$

Minimising the above function finds the maximum likelihood estimates for the parameters in the above equation which are $\beta_0$ and $\beta$. This can be done by differentiating the log likelihood function above with respect to the parameters and setting the partial derivatives equal to 0. But the partial derivatives when set to 0, give rise to a transcendental equation which has no closed form solution. As such, numerical methods are used to solve for maximum likelihood estimates of the parameters. An example numerical method that may be used is Newton's method although other numerical optimisation methods may also be used.

As will be appreciated, in the absence of noise, respiration waveform signals acquired using impedance pneumography tend to be very similar to sine waves, wherein the respiration events i.e. periodic contraction and expansion of the chest during breathing are clearly visible. Thus, a set of basis features such as peaks, troughs and zero crossings become apparent, and hence can be extracted using, for example the methods described in EP2677927.

However, not only these features, but also the number of events of each type and their interval successive differences can provide substantial information about the periodicity of the waveform and the underlying process of respiration itself. Furthermore, if these same events have already been detected for the purposes of determining the respiration rate from the signal, it is advantageous to further use them and various metrics calculated therefrom as feature sets for the logistic regression described above.

In one example, three sets of events of features are extracted from the waveform signal, namely occurrence of peaks, occurrence of troughs and occurrence of zero crossings. These are used as basis events. These basis signal events may be combined using several statistical operators and complexity measures such as the mean, median, mean and median absolute deviation, root mean square standard deviation, entropy, and so on to further populate a full set of features to be used as an input to the logistic regression classifier models. Other features that may also be included in the feature set include wave saturation and turning point ratio of the signal. In this illustrative example, a total of $n$ features are generated in the feature set. Thus, an exemplary continuous respiration signal of a given number of samples per minute may now be represented as a vector of $n$ real discrete values i.e. an $n$-dimensional space.

The features may also be normalised, for example using Gaussian standardisation or range normalisation. Normalisation. Generated features are typically recorded in their raw form - i.e. each feature has numerical values falling into different real number ranges. With different features having different ranges, it is possible that the feature with largest magnitude introduces bias in the decision-making process. Normalisation helps to balance the classification process so that they fall within the same interval.

Continuing with the n-feature set example, in order to build a classifier model that meets the best trade-off between classification performance and low computational complexity, the feature space may be reduced to one of lower dimensionality by performing feature selection. When undertaking this dimensionality reduction, removal of redundant and non-relevant features; and the possibility of visualizing preliminary classification results in context with the feature space data without relying in complex transformations such as Sammon's mapping and PCA may be taken into account.

Example feature selection methods that may be used include: sequential feature selection, where all possible $2^n$ subsets of features of the initial feature set are searched exhaustively, minimum redundancy maximum relevance feature selection, where the relevance of features in the feature subspace are maximised by examining mutual information, and relief technique feature selection.

In the n feature set example, the above described feature selection methods may be used to reduce dimensionality to, for example, a quarter of the number of dimensions although other dimensionality reductions are also envisaged as will be appreciated by the skilled person. In one example, a starting set of 44 features may be reduced to 8 features or further to 3 features, depending on the trade-off between classification and low computational complexity that is desired.

Once a feature set has been selected, the logistic regression classifier model described built thereon using known methods. For example, the *glmfit* function in MATLAB™ may be used.

As will be appreciated by the skilled person, a suitably sized training data set having the chosen feature set may be collected and used to train the model to reach a desired performance level.

Once the model is built and trained, it may be applied to live readings of a monitoring system in a clinical environment to obtain the confidence level associated with the obtained respiration rates.

Accordingly, at this stage two pieces information have been determined: from step 101 the respiration rate value, and from step 102 the result of the classification and confidence level.

The next step is to associate the determined respiration rate and its confidence level with one of a plurality of classes, each having an associated confidence level threshold to be applied to ultimately identify whether the determined respiration rate is valid or invalid.

Some example classes and their details will now be described.

Respiration Rate Classes

Consider where the classes are different respiration rate ranges.

The first range may be a normal respiration rate range for the monitored patient whereas the second respiration rate range may be respiration rate range that is outside the normal range for the patient. For example, an elevated or low respiration rate range.

These ranges may be determined manually by a clinician in advance of using the monitoring system, or may be determined from a series of *n* previous readings of the patient obtained by the monitoring itself. For example, the patient's respiration rate may be measured once or over a period of time where the patient is known to be breathing normally and the range during this time used as that patient's normal range. Alternatively, the normal range may be determined from an average range of a larger cohort of patients. The elevated or low range may then be a percentage increase or decrease from the normal range, for example an increase or decrease of 25%, 50% or more outside the normal range. Alternatively, the elevated range may be determined individually for the monitored patient or patient(s) by collecting respiration rate information while they are known to be breathing at an elevated or low rate.

For example, if the respiration rate is in the normal range and is thus unlikely to require a clinician's attention or be indicative of an adverse physiological event, the first threshold value may be 30% and any respiration rate in this range having a confidence level of 30% or above is considered a valid reading.

However, if the respiration is in the elevated or low range and is thus more likely to require a clinician's attention and be indicative of an adverse physiological event, the second threshold value may be 50% and any respiration rate in this range will need to have a confidence level of 50% or above to be considered a valid reading.

It is also envisaged that additional ranges and associated thresholds may be used to provide a greater degree of granularity in determining whether or not a respiration rate reading is deemed valid or not. For example, if the respiration rate is in a third range, a third threshold value may be applied to the associated confidence level. The third range may be associated with a critical respiration rate of the patient. That is, a respiration rate that is dangerously elevated or low and the patient would require immediate clinical attention if the respiration is a valid reading. In this case, the third threshold may be 75% so that any respiration rate in the critical range will need to have a confidence level of 75% or above to be considered a valid reading.

As above, the critical rate range may be determined as a percentage increase or decrease from the normal range, for example a 75%-100%, or more increase or decrease outside the normal range, or may be determined from previous measurements of the patient or cohort of patients.

If a reading in the critical range is deemed valid, a warning or alarm may be given to the clinician to notify them of a potentially dangerous adverse physiological event.

The above three-threshold system may accordingly be summarised with the following pseudocode:

- if (RR value = normal range), report RR value if SQI > 30%
- if (RR value = warning range), report RR value if SQI > 50%
- if (RR value = critical range), report RR value if SQI > 75%

Whereby RR is respiration rate and SQI is signal quality indicator or confidence level.

In this way, only high quality, low noise signals with high confidence scores will result warnings and alarms while lower quality, higher noise signals can continue to be used to ensure continuous monitoring of respiration rates is maintained.

Classes having expected or unexpected readings based on prior readings

Consider where the classes are readings having an expected value or an unexpected value based on previous readings.

If the new respiration rate value is similar to prior data (e.g. within a few breaths per minute of the mean of the previous 15 minutes), then the new data is "expected". In this case, a lower confidence level threshold can be applied. Alternatively if the new data is very different to the previous values (i.e. it is "unexpected") then it could be an outlier and so the confidence level threshold may be raised.

In order to make this determination, a Kalman filter may be applied to previously determined respiration rates, incoming respiration rates, and/or other inputs including statistical noise and other inaccuracies to predict the expected respiration rate. A comparison between the expected rate or range of expected rates predicted by the Kalman filter and the incoming rate may then be performed and the unexpected rate class boundaries set to be a predetermined deviation from the expected rate. For example, if the incoming respiration rate deviates 5%, 10%, 15% or more from the rate predicted by the Kalman filter then the incoming rate is associated with the unexpected class whereas if it deviates less than this amount than it is associated with the expected class.

It will be appreciated that actual rate value of the boundary at which a reading is considered to fall within the expected or unexpected class may change as the data used constantly changes to the most recent set of readings which itself is constantly changing.

It will also be appreciated that a Kalman filter is only one way of predicting an expected rate and determining where the class boundary between the expected and unexpected rate class is set. Other predictive models may also be used.

Patient Activity Level Classes

Consider where the classes are patient activity levels.

A patient's physical activity level may be measured using an accelerometer attached to the patient. For example, the accelerometer may be incorporated into the same device such as a patch in which the impedance pneumography device is provided.

Respiration rate readings obtained while a patient has a high activity level are likely to be inaccurate and suffer from motion artefacts. In such a case, the patch records accelerometer data during the recorded respiration episode and this data may be fed into a classifier of the monitoring system. Such a classifier may be trained to classify accelerometer data for a given time period into one of a number of activity level classes e.g. the classes of no activity, low activity or moderate/high activity. The respiration rate determined from the waveform signal corresponding to that same time period may then be associated with that class. The likelihood of motion artefacts affecting the determination of the respiration rate during low activity levels is small so a low confidence level threshold may be applied. Conversely, higher confidence level thresholds may be applied to higher activity levels.

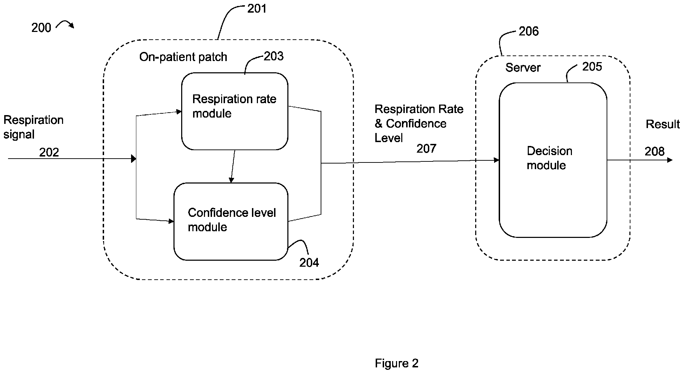

Figure 2 shows a block diagram of a respiration monitoring system 200. The system comprises a body-worn sensor 201, for example an impedance pneumography device, configured to record a waveform signal 202 representative of a respiration episode.

The system 200 may further comprise a one or more processors and a transmitter (not-shown) co-located with the body-worn sensor 201. The transmitter is configured to transmit one or more outputs of the system (e.g. respiration rate, confidence level) to a server remote from the body-worn sensor, for example a server of a central computer system of a clinical environment.

The co-located processor(s), body-warn sensor 201,(and where applicable the transmitter) may be configured as part of a wearable wireless device and/or low-power battery operated disposable device such as a patch that may be secured to a patient for continuous monitoring in a clinical environment.

Referring back to Figure 2, the system 200 further comprises a respiration rate module 203 and a confidence level module 204 configured respectively to determine a respiration rate and associated confidence level from the recorded respiration signal 202.

The respiration rate module 203 and confidence level module 204 may be implemented using the one or more processors. For example, the respiration rate module 203 and confidence level module 204 may be implemented on one or more of the processors co-located with the body-worn sensor 201 and configured as a wearable and/or low-power disposable device. Alternatively, one or both of modules 203, 204 may instead be implemented on one or more processors remote from the body-worn sensor 201, for example on a server. As will be appreciated, the above-described rules-based approach to determining the respiration rate, and the application of the logistic regression model (once trained) to input data is computationally relatively inexpensive thus allowing these to be applied by such wearable, low-powered battery operated, disposable devices. It will be further be appreciated, that the building and training of the models is performed separately on a device having substantially higher computational resources available to it, for example on a central server or cloud system.

The system in Figure 2 further comprises a decision module 205 configured to apply the threshold values to the confidence level according to the class to which the respiration rate is assigned. For example, this may comprise matching the assigned class with a list of stored classes and stored associated thresholds and applying the applicable threshold to the confidence level when a match is found. With the thresholds applied, the decision module 205 is configured to output 208 a valid respiration rate result or error code or message if the confidence level is not high enough to exceed the applied threshold.

It is envisaged that the decision module 205 is implemented on one or more processors of a centralised device such as a server 206 whereby the respiration rates and confidence levels 207 are transmitted from the body-worn sensor 201 and co-located processor(s) to the server 206 using one or more known wireless technologies including for example Bluetooth™, WiFi™ and the like.

An advantage of implementing the decision module 205 on a processor in the server 206 instead of in the on-patient part of the system 200 is that it is not necessary to store the recorded respiration rate values, confidence levels, threshold levels and other data in the on-patient part of the system such as a patch. Thus the memory and associated power requirements of the on-patient part of the system are reduced, giving the on-patient part of the system 300 a longer battery life and use time before needing to be replaced.

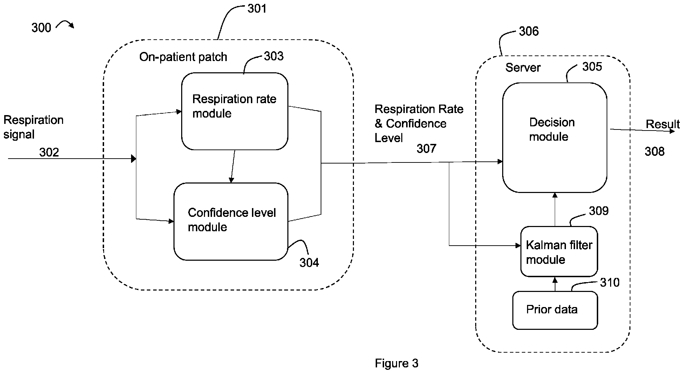

Figure 3 shows a block diagram of a respiration monitoring system 300 that is similar to the system 200 of Figure 2. The numbered features 301-308 in Figure 3 correspond to the features 201-208 in Figure 2. Additionally, the system 300 comprises a Kalman filter module 309 which is fed with prior respiration rate data 310 to provide a predicted respiration rate with which to set the boundary value of the expected or unexpected classes. The respiration rate may then be compared to the class boundary and associated with the applicable class. The decision module 305 may then apply the applicable threshold for that class to the confidence level to determine if the respiration rate is valid or not. The Kalman filter module 309 and the associated storage of prior data 310 in Figure 3 are implemented on one or more processors and memory of a server 306 remote from the on-patient part of the system. This again ensures that the memory and associated power requirements of the on-patient part of the system are reduced.

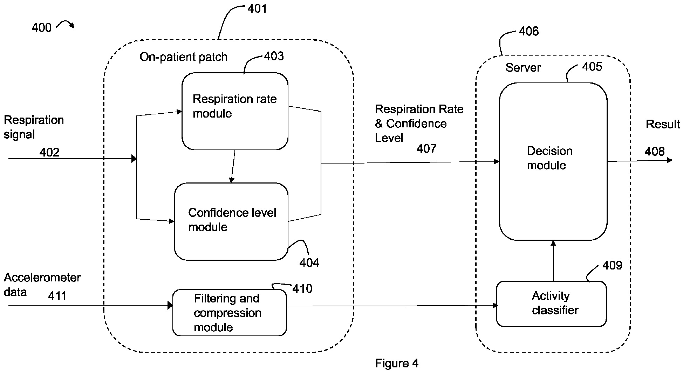

Figure 4 shows a block diagram of a respiration monitoring system 400 that is similar to the system 200 of Figure 2. The numbered features 401-408 in Figure 3 correspond to the features 201-208 in Figure 2. Additionally, the system 400 in Figure 4 comprises an activity classifier 409 and an optional filtering and compression module 410. The activity classifier 409 is implemented on one or more processors on the server 406 and receives accelerometer data 411 from an accelerometer (not shown) that forms part of the on-patient part of the system. The received accelerometer data 411 is classified into an activity level class and the respiration rate for the corresponding period is associated with that class. The decision module 405 may then apply the confidence level threshold associated with that class to the confidence level of the respiration rate to determine if it is valid or not.

As the volume of accelerometer data 411 if captured continuously may be substantial and could result in increased power consumption at the on-patient part of the system if it were all to be transmitted to the server 406, the filtering and compression module 410 is used to clean and compress the data 411 so that the total volume of transmitted data is reduced.

For example, features described in relation to one embodiment may be incorporated into another embodiment or vice versa.

For example, whilst the present description envisages using the one or more methods described in EP2677927 to determine respiration rate, it will be appreciated that other known methods of determining respiration rates may be used instead.

For example, whilst three different respiration rate range classes and associated thresholds are described herein, it is also envisaged that other numbers of range classes and thresholds may be used to increase the granularity of the monitoring system, for example five, six, seven or more respiration ranges and associated confidence level thresholds may also be used.

For example, the decision module described herein may receive input from multiple sources including multiple classifiers based on e.g. ranges, statistics, activity levels and so on to determine what class or classes the respiration rate is in and accordingly which threshold or thresholds to apply to determine the validity of the respiration rate. Accordingly, the activity level, range and Kalman filter examples herein are illustrative only.

For example, one or more pre-processing steps may be applied to the raw respiration waveform signal in the on-patient part of the system before the respiration rate is determined therefrom. Two particular pre-processing steps are particularly effective at increasing respiration rate accuracy, namely: (i) applying a high-pass and/or low-pass filter to remove high frequency noise (such as high-frequency artefacts, and myogenic noise) and low frequency baseline wander respectively; and (ii) applying a notch filter to remove heart bumps. Heart bumps are events introduced in the respiration signal by the beating of the heart whereby the notch filter may use a measured heart rate of the patient as an input. The notch filter may be, for example, a tuneable bi-quad notch filter whereby the filter positions the "notch" at the frequency corresponding to the heart rate, attenuating the heart bumps in the signal. In the event a heart rate value of the patient is not available, the notch filter may be automatically switched off for that particular period.

Finally, one or more derivatives of the respiration signal such as the first order derivative may be calculated which may be used in the peak/trough detection when determining the respiration rate. Thus, the rule-based peak/trough detection uses the input filtered signal, as well as its first-order derivative, to identify individual peaks and troughs in sequence. And, as described in EP2677927, each detection is subjected to a number of rules in real-time, including a slope threshold rule, a dynamic amplitude rule, and a peak/trough sequence rule. If accepted, each detection is stored in a memory of the system, and the process continues until the end of the respiration signal is reached. The signal is assessed for periodicity using the information from both peak and trough detections. If no periodicity information can be extracted from the signal, an invalid result indication may be given in the same way that would occur when a confidence level is below the applied threshold.

**Claims**

1. A computer-implemented method of identifying a validly determined respiration rate, the method comprising:

   determining (101,102) a respiration rate and a confidence level associated with the respiration rate from a waveform signal representative of a recorded respiration episode;
   associating (103) the respiration rate with one of at least first and second classes, the classes being associated with respective different confidence level thresholds;
   wherein at least one of the classes is a first range of respiration rates,
   wherein at least one of the classes is a second range of respiration rates,
   wherein the first range comprises a normal respiration rate range of a patient and the second range comprises a respiration rate range outside said normal respiration rate range and
   wherein the confidence level threshold associated with the first class is lower than that associated with the second class and
   if (106) the confidence level exceeds a confidence level threshold associated with the class with which the respiration rate is associated, determining (107a) that the respiration rate is valid and outputting the determined respiration rate; or
   if (106) the confidence level is below the confidence level threshold associated with the class with which the respiration rate is associated, determining (107b) that the respiration rate is invalid and outputting an invalid result notification.

2. The method of claims 1, wherein determining the confidence level comprises applying a logistic regression-based model to the waveform signal.

3. The method of any preceding claim, wherein determining the respiration rate comprises applying a rules-based model to the waveform signal to identify peaks and troughs in the waveform signal, and determining the respiration rate from a periodicity of the identified peaks and troughs.

4. The method of any of claims 1 to 3, wherein at least one of the classes is a first physical activity level of the patient, wherein at least one of the classes is a second physical activity level of the patient, and wherein said associating the respiration rate with the one of at least first and second classes comprises:

   applying a classifier to accelerometer data captured from the patient during said respiration episode to classify the patient's physical activity during said respiration episode into said first or second activity level classes, and associating the respiration rate of said respiration episode with said first or second activity level classes.

5. The method of any of claims 1 to 3, wherein at least one of the classes is an expected respiration rate range, wherein at least one of the classes is an unexpected respiration rate range, and wherein said associating the respiration rate with the one of at least first and second classes comprises:

   applying a Kalman filter to previously determined respiration rates to determine a range of expected and unexpected respiration rates, and
   determining if the respiration rate is in the expected or unexpected respiration rate range.

6. One or more processors for a respiration monitoring system, the one or more processors configured for:

(i) determining a respiration rate and a confidence level associated with the respiration rate from a waveform signal representative of a recorded respiration episode;

(ii) associating the respiration rate with one of at least first and second classes, the classes being associated with respective different confidence level thresholds wherein at least one of the classes is a first range of respiration rates, wherein at least one of the classes is a second range of respiration rates, wherein the first range comprises a normal respiration rate range of a patient and the second range comprises a respiration rate range outside said normal respiration rate range and wherein the confidence level threshold associated with the first class is lower than that associated with the second class; and

(iii) if the confidence level exceeds a confidence level threshold associated with the class with which the respiration rate is associated, determining that the respiration rate is valid and outputting the determined respiration rate; or

(iv) if the confidence level is below the confidence level threshold associated with the class with which the respiration rate is associated, determining that the respiration rate as invalid and outputting an invalid result notification.

7. A respiration monitoring system comprising:

a body-worn sensor (201) for recording a waveform signal representative of a respiration episode; and
one or more processors according to claim 6.

8. The system of claim 7, wherein the one or more processors performing the steps of determining the respiration rate and determining of the confidence level are co-located with the body-worn sensor (201).

9. The system of claim 7 or 8 comprising an accelerometer co-located with the body-worn sensor and configured to record accelerometer data during said respiration episode.

10. The system of any of claims 7 to 9 and comprising a transmitter co-located with the body-worn sensor (201) and configured to transmit the determined respiration rate and confidence level, and where applicable accelerometer data, to a server remote (206) from the body-worn sensor.

11. The system of any of claims 7 to 10, wherein the body-worn sensor (201), the one or more co-located processors and where applicable the co-located transmitter and co-located accelerometer are configured as a wearable wireless device and/or a low-power battery operated disposable device.


**Patentansprüche**

1. Computerimplementiertes Verfahren zum Identifizieren einer gültig bestimmten Atmungsrate, wobei das Verfahren Folgendes umfasst:

Bestimmen (101, 102) einer Atmungsrate und eines der Atmungsrate zugeordneten Vertrauensniveaus anhand eines Wellenformsignals, das eine aufgezeichnete Atmungsepisode darstellt;
Zuordnen (103) der Atmungsrate zu einer von mindestens einer ersten und einer zweiten Klasse, wobei die Klassen jeweils unterschiedlichen Vertrauensniveau-Schwellenwerten zugeordnet sind,
wobei mindestens eine der Klassen ein erster Bereich von Atmungsraten ist,
wobei mindestens eine der Klassen ein zweiter Bereich von Atmungsraten ist,
wobei der erste Bereich einen normalen Atmungsratenbereich eines Patienten umfasst und der zweite Bereich einen Atmungsratenbereich außerhalb des normalen Atmungsratenbereichs umfasst und
wobei der der ersten Klasse zugeordnete Vertrauensniveau-Schwellenwert niedriger ist als der der zweiten Klasse zugeordnete und
wenn (106) das Vertrauensniveau einen Vertrauensniveau-Schwellenwert überschreitet, welcher der Klasse zugeordnet ist, der die Atmungsrate zugeordnet ist, Bestimmen (107a), dass die Atmungsrate gültig ist, und Ausgeben der bestimmten Atmungsrate; oder
wenn (106) das Vertrauensniveau unter dem Vertrauensniveau-Schwellenwert liegt, welcher der Klasse zugeordnet ist, der die Atmungsrate zugeordnet ist, Bestimmen (107b), dass die Atmungsrate ungültig ist, und Ausgeben einer Meldung über ein ungültiges Ergebnis.

2. Verfahren nach Anspruch 1, wobei das Bestimmen des Vertrauensniveaus das Anwenden eines auf logistischer Regression basierenden Modells auf das Wellenformsignal umfasst.

3. Verfahren nach einem der vorhergehenden Ansprüche, wobei das Bestimmen der Atmungsrate das Anwenden eines regelbasierten Modells auf das Wellenformsignal, um Spitzen und Täler in dem Wellenformsignal zu identifizieren, und das Bestimmen der Atmungsrate aus einer Periodizität der identifizierten Spitzen und Täler umfasst.

4. Verfahren nach einem der Ansprüche 1 bis 3, wobei mindestens eine der Klassen ein erstes körperliches Aktivitätsniveau des Patienten ist, wobei mindestens eine der Klassen ein zweites körperliches Aktivitätsniveau des Patienten ist, und wobei das Zuordnen der Atmungsrate zu der einen von mindestens einer ersten und einer zweiten Klasse Folgendes umfasst:

Anwenden eines Klassifizierers auf Beschleunigungsmesserdaten, die von dem Patienten während der Atmungsepisode erfasst werden, um die körperliche Aktivität des Patienten während der Atmungsepisode in die erste oder zweite Aktivitätsniveauklasse einzustufen, und
Zuordnen der Atmungsrate der Atmungsepisode zu der ersten oder der zweiten Aktivitätsniveauklasse.

5. Verfahren nach einem der Ansprüche 1 bis 3, wobei mindestens eine der Klassen ein Bereich erwarteter Atmungsraten ist, wobei mindestens eine der Klassen ein Bereich unerwarteter Atmungsraten ist, und wobei das Zuordnen der Atmungsrate zu der einen von mindestens einer ersten und einer zweiten Klasse Folgendes umfasst:

Anwenden eines Kalman-Filters auf zuvor bestimmte Atmungsraten, um einen Bereich von erwarteten und unerwarteten Atmungsraten zu bestimmen, und
Bestimmen, ob die Atmungsrate in dem erwarteten oder dem unerwarteten Atmungsratenbereich liegt.

6. Prozessor(en) für ein Atmungsüberwachungssystem, wobei der eine oder die mehreren Prozessoren zu Folgendem konfiguriert ist/sind:

(i) Bestimmen einer Atmungsrate und eines der Atmungsrate zugeordneten Vertrauensniveaus anhand eines Wellenformsignals, das eine aufgezeichnete Atmungsepisode darstellt;
(ii) Zuordnen der Atmungsrate zu einer von mindestens einer ersten und einer zweiten Klasse, wobei die Klassen jeweils unterschiedlichen Vertrauensniveau-Schwellenwerten zugeordnet sind, wobei mindestens eine der Klassen ein erster Bereich von Atmungsraten ist, wobei mindestens eine der Klassen ein zweiter Bereich von Atmungsraten ist, wobei der erste Bereich einen normalen Atmungsratenbereich eines Patienten umfasst und der zweite Bereich einen Atmungsratenbereich außerhalb des normalen Atmungsratenbereichs umfasst und wobei der der ersten Klasse zugeordnete Vertrauensniveau-Schwellenwert niedriger ist als der der zweiten Klasse zugeordnete; und
(iii) wenn das Vertrauensniveau einen Vertrauensniveau-Schwellenwert überschreitet, welcher der Klasse zugeordnet ist, der die Atmungsrate zugeordnet ist, Bestimmen, dass die Atmungsrate gültig ist, und Ausgeben der bestimmten Atmungsrate; oder
(iv) wenn das Vertrauensniveau unter dem Vertrauensniveau-Schwellenwert liegt, welcher der Klasse zugeordnet ist, der die Atmungsrate zugeordnet ist, Bestimmen, dass die Atmungsrate ungültig ist, und Ausgeben einer Meldung über ein ungültiges Ergebnis.

7. System zur Überwachung der Atmung, Folgendes umfassend:

einen am Körper getragenen Sensor (201) zum Aufzeichnen eines Wellenformsignals, das eine Atmungsepisode darstellt; und
einen oder mehrere Prozessoren nach Anspruch 6.

8. System nach Anspruch 7, wobei der eine oder die mehreren Prozessoren, welche die Schritte des Bestimmens der Atmungsrate und des Bestimmens des Vertrauensniveaus durchführen, gemeinsam mit dem am Körper getragenen Sensor (201) angeordnet sind.

9. System nach Anspruch 7 oder 8, umfassend einen Beschleunigungsmesser, der mit dem am Körper getragenen Sensor gemeinsam angeordnet und konfiguriert ist, um Beschleunigungsmesserdaten während der Atmungsepisode aufzuzeichnen.

10. System nach einem der Ansprüche 7 bis 9, und umfassend einen Sender, der gemeinsam mit dem am Körper getragenen Sensor (201) angeordnet und konfiguriert ist, um die bestimmte Atmungsrate und das bestimmte Vertrauensniveau sowie gegebenenfalls Beschleunigungsmesserdaten an einen vom am Körper getragenen Sensor entfernten Server (206) zu übertragen.

11. System nach einem der Ansprüche 7 bis 10, wobei der am Körper getragene Sensor (201), der eine oder die mehreren gemeinsam angeordneten Prozessoren und, gegebenenfalls, der gemeinsam angeordnete Sender und der gemeinsam angeordnete Beschleunigungsmesser als eine tragbare drahtlose Vorrichtung und/oder als eine batteriebetriebene Einwegvorrichtung mit geringem Stromverbrauch konfiguriert sind.

## Revendications

1. Procédé mis en oeuvre sur ordinateur permettant d'identifier une fréquence respiratoire déterminée de façon valide, le procédé comprenant :

   la détermination (101, 102) d'une fréquence respiratoire et d'un niveau de confiance associé à la fréquence respiratoire à partir d'un signal de forme d'onde représentatif d'un épisode de respiration enregistré ;
   l'association (103) de la fréquence respiratoire à une catégorie parmi au moins une première et une deuxième catégorie, les catégories étant associées à des seuils respectifs différents de niveau de confiance ;
   dans lequel au moins l'une des catégories est une première gamme de fréquences respiratoires,
   dans lequel au moins l'une des catégories est une deuxième gamme de fréquences respiratoires,
   dans lequel la première gamme comprend une gamme de fréquences respiratoires normales d'un patient et la deuxième gamme comprend une gamme de fréquences respiratoires en dehors de ladite gamme de fréquences respiratoires normales, et
   dans lequel le seuil de niveau de confiance associé à la première catégorie est inférieur à celui associé à la deuxième catégorie, et
   si (106) le niveau de confiance dépasse un seuil de niveau de confiance associé à la catégorie à laquelle la fréquence respiratoire est associée, la détermination (107a) que la fréquence respiratoire est valide et la sortie de la fréquence respiratoire déterminée ; ou
   si (106) le niveau de confiance est inférieur au seuil de niveau de confiance associé à la catégorie à laquelle la fréquence respiratoire est associée, la détermination (107b) que la fréquence respiratoire est invalide et la sortie d'une notification de résultat invalide.

2. Procédé selon la revendication 1, dans lequel la détermination du niveau de confiance comprend l'application d'un modèle basé sur une régression logistique au signal de forme d'onde.

3. Procédé selon l'une quelconque des revendications précédentes, dans lequel la détermination de la fréquence respiratoire comprend l'application d'un modèle basé sur des règles au signal de forme d'onde pour identifier des crêtes et des creux dans le signal de forme d'onde, et la détermination de la fréquence respiratoire à partir d'une périodicité des crêtes et creux identifiés.

4. Procédé selon l'une quelconque des revendications 1 à 3, dans lequel au moins l'une des catégories est un premier niveau d'activité physique du patient, dans lequel au moins l'une des catégories est un deuxième niveau d'activité physique du patient, et dans lequel ladite association de la fréquence respiratoire à ladite une d'au moins une première et une deuxième catégorie comprend :

   l'application d'un classificateur à des données d'accéléromètre capturées à partir du patient pendant ledit épisode de respiration pour classifier l'activité physique du patient pendant ledit épisode de respiration en ladite première ou deuxième catégorie de niveau d'activité, et
   l'association de la fréquence respiratoire dudit épisode de respiration à ladite première ou deuxième catégorie de niveau d'activité.

5. Procédé selon l'une quelconque des revendications 1 à 3, dans lequel au moins l'une des catégories est une gamme de fréquences respiratoires attendues, dans lequel au moins l'une des catégories est une gamme de fréquences respiratoires non attendues, et dans lequel ladite association de la fréquence respiratoire avec ladite une d'au moins une première et une deuxième catégorie comprend :

   l'application d'un filtre de Kalman à des fréquences respiratoires déterminées précédemment pour déterminer une gamme de fréquences respiratoires attendues et non attendues, et
la détermination si la fréquence respiratoire est dans la gamme de fréquences respiratoires attendues ou non attendues.

6. Processeur(s) pour un système de surveillance respiratoire, les un ou plusieurs processeurs étant configurés pour :

i) déterminer une fréquence respiratoire et un niveau de confiance associé à la fréquence respiratoire à partir d'un signal de forme d'onde représentatif d'un épisode de respiration enregistré ;
ii) associer la fréquence respiratoire à l'une d'au moins une première et une deuxième catégorie, les catégories étant associées à des seuils respectifs différents de niveau de confiance, dans lequel au moins une des catégories est une première gamme de fréquences respiratoires, dans lequel au moins une des catégories est une deuxième gamme de fréquences respiratoires, dans lequel la première gamme comprend une gamme de fréquences respiratoires normales d'un patient et la deuxième gamme comprend une gamme de fréquences respiratoires en dehors de ladite gamme de fréquences respiratoires normales, et dans lequel le seuil de niveau de confiance associé à la première catégorie est inférieur à celui associé à la deuxième catégorie ; et
iii) si le niveau de confiance dépasse un seuil de niveau de confiance associé à la catégorie à laquelle la fréquence respiratoire est associée, déterminer que la fréquence respiratoire est valide et émettre en sortie la fréquence respiratoire déterminée ; ou
iv) si le niveau de confiance est inférieur au seuil de niveau de confiance associé à la catégorie à laquelle la fréquence respiratoire est associée, déterminer que la fréquence respiratoire est invalide et émettre en sortie une notification de résultat invalide.

7. Système de surveillance de respiration comprenant :

un capteur porté sur le corps (201) pour enregistrer un signal de forme d'onde représentatif d'un épisode de respiration ; et
un ou plusieurs processeurs selon la revendication 6.

8. Système selon la revendication 7, dans lequel les un ou plusieurs processeurs effectuant les étapes de détermination de la fréquence respiratoire et de détermination du niveau de confiance sont co-implantés avec le capteur porté sur le corps (201).

9. Système selon la revendication 7 ou 8 comprenant un accéléromètre co-implanté avec le capteur porté sur le corps et configuré pour enregistrer des données d'accéléromètre pendant ledit épisode de respiration.

10. Système selon l'une quelconque des revendications 7 à 9 et comprenant un émetteur co-implanté avec le capteur porté sur le corps (201) et configuré pour transmettre la fréquence respiratoire et le niveau de confiance déterminés, et, le cas échéant, des données d'accéléromètre, à un serveur à distance (206) du capteur porté sur le corps.

11. Système selon l'une quelconque des revendications 7 à 10, dans lequel le capteur porté sur le corps (201), les un ou plusieurs processeurs co-implantés et, le cas échéant, l'émetteur co-implanté et l'accéléromètre co-implanté sont configurés sous forme d'un dispositif portable sans fil et/ou d'un dispositif à usage unique alimenté par pile à consommation faible d'énergie.

100

101 | Determine a respiration rate from a waveform signal representative of a recorded respiration episode |

102 | Determine a confidence level associated with the respiration rate |

103 | Associate respiration rate with one of at least first and second classes |

104 | First class or second class? |

105a | First class associated with first threshold |

105b | Second class, associated with second threshold |

106 | Does confidence level exceed threshold? |

107a | Yes, determine respiration rate is valid and output respiration rate |

107b | No, determine respiration rate is invalid and output invalid reading notification |

Figure 1

200

201

On-patient patch

203

Respiration rate
module

Respiration
signal

202

Confidence level
module

204

Respiration Rate
& Confidence
Level

207

206

Server

205

Decision
module

Result

208

Figure 2

Figure 3

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1928311 A
- EP 2677927 A
- US 20200163586 A1